Europäisches Patentamt

European Patent Office

Office européen des brevets

(19)

(11) Publication number: **0 008 473**
**B1**

(12) **EUROPEAN PATENT SPECIFICATION**

(45) Date of publication of patent specification: **01.06.83**

(51) Int. Cl.³: **G 01 N 33/58**

(21) Application number: **79200440.0**

(22) Date of filing: **10.08.79**

(54) **Method for the detection and/or determination of an antigen specific immunoglobulin, immuno-reagents to be used in said method and test kit for said determination.**

(30) Priority: **24.08.78 NL 7808729**

(43) Date of publication of application:
**05.03.80 Bulletin 80/5**

(45) Publication of the grant of the patent:
**01.06.83 Bulletin 83/22**

(84) Designated Contracting States:
**BE CH DE FR GB IT LU NL SE**

(56) References cited:
**NL - A - 7 501 215**

**JOURNAL OF IMMUNOLOGY, vol. 116, no. 6, june 6, 1976 The Williams & Wilkins Co. Baltimore US K. KATO et al.: "Enzyme-linked immunoassay: Conjugation of the FAB'-fragment of Rabbit IgG with beta-D-Galactosidase from E. Coli and its use for immunoassay", pages 1554—1560
Essential Immunology—I. Roitt-4th Edn. pp 148—151**

The file contains technical information submitted after the application was filed and not included in this specification

(73) Proprietor: **Akzo N.V.**
**Velperweg 76**
**NL-6824 BM Arnhem (NL)**

(72) Inventor: **Duermeyer, Willem**
**Sibeliuslaan 64**
**Oss (NL)**
Inventor: **van den Akker, Rudolf**
**Oude Brandenburgerweg 10**
**Bilthoven (NL)**

(74) Representative: **Douma, Anno Do et al,**
**Postbus 20**
**NL-5340 BH Oss (NL)**

Method for the detection and/or determination of an antigen specific immunoglobulin,
immuno-reagents to be used in said method and test kit for said determination

The invention relates to a method for the detection and/or determination of an antigen specific immunoglobulin, to a reagent and to test kits for use in such determinations.

The immunoglobulins can be subdivided into five classes G, A, M, D and E. Immunoglobulins of these classes are indicated with IgG, IgA, IgM, IgD and IgE respectively. In the following, immunoglobulins of a certain class will be indicated with IgX, in which X means G, A, M, D and E.

Immunoglobulins are structurally related and contain at least two heavy (long) chains (H-chains) and two light (short) chains (L-chains), which are mutually connected by disulphide bridges and sometimes via additional polypeptides. The heavy and light chains each have a variable and a constant region. Some immunoglobulins consist of a multiple of the basic structure of two heavy and two light chains.

Antibodies are immunoglobulins, which can bind antigens specifically. The antibody specificity is located in the variable regions of the 4 peptide chains, which are all situated at the same side of the molecule (N-terminal side). A number of biological actions of a certain antibody is mediated through the constant regions of the chains of the immunoglobulin molecule concerned.

Antibodies can be cleaved into fragments, which still have antigen specificity, and crystallizable fragments without this antigen binding property. Antigen binding fragments are e.g. Fab-, Fab'- and F(ab')$_2$-fragments. Crystallizable fragments without antigen specificity are e.g. Fc- and Fc'-fragments.

The concentration of immunoglobulins of the several classes in normal human serum is: IgG 8—16 mg/ml, IgA 1.4—4 mg/ml, IgM 0.5—2.0 mg/ml, IgD 0.0—0.4 mg/ml and IgE 0.000017—0.000450 mg/ml.

Quantitatively antibodies of the IgG class constitute therefore the most important group of antibodies. Antibodies of the IgM class are present in the early stages of an infection, so that determination of antibodies of the IgM class is very important for the early detection of an infectious disease.

Antibodies of the IgA, IgD and IgE classes can be present in serum in increased concentration in certain pathological conditions. For example, antibodies of the IgE class are present in increased concentrations in allergic conditions and antibodies of the IgD class are involved in auto-immune diseases.

The determination of antigen specific immunoglobulins of a peculiar class is of particular clinical significance. Antigen specific immunoglobulins can be determined with immunochemical techniques, in which use is made of an immuno-component with binding affinity to the antibody to be detected and/or determined. According to a known technique, an immuno component with binding affinity to the antibody to be determined is made insoluble by coupling to a solid carrier and another specific bindable substance is labelled, for example with a fluorescent, chromophoric or radio-active group or with an enzyme.

This known technique then proceeds by mixing a test medium with the insolubilized immuno-component and after incubation add the labelled immuno-component. After a second incubation the label is detected either in the insoluble phase or in the liquid phase. This technique is employed in histochemistry for the detection of antibodies to specific cells. The serum to be investigated is mixed with a sample of gastric parietal cells. Antibodies to these gastric cells present in the serum will adhere to the surface of the cells and are detectable after subsequent binding of a label. According to another method antibodies can be detected by binding them to insolubilized antibodies specific to the antibody to be detected. This immuno-complex is reacted with labelled antigen binding fragments of antibodies specific to the antibody to be detected. A disadvantage of these techniques is, that if the rheumatoid factor (RF) is present, which is often present in serum, false positive reactions may be obtained.

Furthermore, the methods for separation of immunoglobulins of different classes and especially of antigen specific immunoglobulins of different classes are elaborate and time consuming and give generally only qualitative or semi-quantitative results. Examples of these methods are immuno-diffusion, immuno-electrophoresis and sucrose density gradient centrifugation.

The rheumatoid factor which often causes false-positive results, is itself also an immunoglobulin, usually of the IgM class. The rheumatoid factor (RF) has affinity for antibodies of the IgG class. RF binds via the constant regions of the heavy chains of the IgG molecule and especially that part, which upon cleavage of the antibody is separated from the antigen binding fragments. Because the rheumatoid factor is usually of the IgM class it will also be bound by anti-IgM immunoglobulins.

The invention now relates to a method for the determination of an antigen specific immunoglobulin of a peculiar IgX class, in which interference of the rheumatoid factor is avoided.

According to the method of the invention, the serum in which an antigen specific immunoglobulin of a peculiar IgX class is to be detected and/or determined, is mixed with an insolubilized anti-IgX against the antigen specific IgX concerned or an antigen binding fragment of this anti-IgX with which the particular IgX to be determined is allowed to react. An incubation is subsequently performed with an antigen for which the immunoglobulin has specific affinity, after which a further incubation takes place with a labelled antigen binding fragment of an antibody against the above-noted antigen.

The fragmentation of an antibody into crystallizable and antigen binding fragment or fragments may be effected by enzymatic cleavage of the heavy chains or by enzymatic cleavage of these chains and subsequent chemical cleavage of the obtained antigen binding fragment into two smaller antigen binding fragments.

For example, by enzymatic cleavage with papain, two identical monovalent antigen binding Fab-fragments and a crystallizable Fc-fragment without antigen binding properties are obtained. By enzymatic cleavage with pepsine a divalent antigen binding F(ab')$_2$-fragment is obtained, which can be chemically cleaved into two Fab'-fragments.

Although RF is possibly bound by the insolubilized anti-IgX and may be bound directly or indirectly by insolubilized antigen binding fragment of this anti-IgX, a labelled antigen binding fragment of an antibody will not be bound by the RF. In this way false-positive reactions due to the RF will be avoided. The incubation with antigen and the subsequent incubation with labelled antibody fragment may also be performed in one step by using as reagent a previously labelled antigen, whereby labelling has been effected by coupling this antigen with the labelled antibody fragment by methods known *per se* for coupling antigens and antibodies in immunological test reactions. The invention therefore also relates to these reagents, consisting of a coupling product of an antigen with a labelled antigen binding part of an antibody against this antigen. By use of this reagent, intensive purification of the antigen is not necessary, which is a substantial advantage.

As stated above, the method according to the present invention may be used for qualitative and quantitative determination of an antigen specific immunoglobulin of each of the five IgX classes separately, without the occurrence of false-positive reactions due to the rheumatoid factor.

The solid carrier to which the anti-IgX or antigen binding fragment of this anti-IgX is coupled may be any water-insoluble solid carrier where coupling is effected by means of covalent bonds or by means of adsorption. The solid carrier may be in the form of granules or strips of various shapes and size. It may also be a tube or a micro-titration plate, in which the immunochemical reaction is performed, whereby the anti-IgX component is coupled to the inner surface of the reaction vessel.

The antigen binding fragment of the antibody with affinity to the antigen to be used may be labelled with enzymes or with fluorescent, chromophoric or radio-active groups or atoms. Use is preferably made of enzyme labelling. Examples of enzymes include catalase, peroxidase, urease, glucose oxidase and phosphatase, but many other enzymes are possible. After conclusion of the immunochemical reaction, an enzyme substrate is added to the liquid and/or solid phase of the reaction mixture obtained, after which an enzyme determination is performed, for example colorimetrically, fluorimetrically or spectrophotometrically.

The invention also relates to test kits, to be used in the method according to the determination.

The test kit is composed predominantly of the following components:

a) a certain quantity of an insolubilized anti-IgX or antigen binding fragment of this anti-IgX,
b) a certain quantity of an antigen, against which the Igx to be determined is directed,
c) a certain amount of a labelled antigen binding fragment of an antibody against the antigen referred to in (b).

The labelled antibody fragment referred to in (c) may be labelled with an enzyme.

In that case, the test kit also contains a substrate for the determination of the amount of the enzyme used.

Instead of the separate components (b) and (c), the test kit may also contain a single reagent, namely a coupling product of an antigen with a labelled fragment of an antibody against this antigen.

The invention is further illustrated by means of the following examples.

Example I
Determination of IgM anitbodies against hepatitis A virus/antigen.

A. Preparation of animal anti-human IgM
Rabbit anti-human IgM was prepared according to a method described by R. Gispen, J. Nagel, B. Brand-Saathof and S. De Graaf, Clin. Exp. Immunol. *22*, 1975, 431—437.

The specificity of the anti-IgM was increased by absorbing the anti-IgM serum with human umbilical cord serum for removal of anti-IgG.

0.05 ml umbilical cord serum was added to 0.2 ml anti-IgM serum and the mixture was incubated for 1 hour at 37°C. It was then centrifuged at 14000 g for 20 minutes. The supernatant was kept at 4°C.

B. Preparation of hepatitis A virus/antigen
A 20% extract was prepared from a faecal sample containing hepatitis A. 1 g faeces was mixed with 4 ml 0.005 M phosphate buffer, pH 7.2 (PBS) and 4 ml chloroform, and the whole was shaken vigorously for 5 minutes. The suspension was then centrifuged at 3000 g for 30 minutes at 4°C. The aqueous phase was pipetted off and kept at −20°C. When necessary, an amount of 20% extract was diluted in PBS such that an extinction of about 1.00 was obtained with the diluted extract in the sandwich enzyme immuno-assay for the determination of hepatitis A antigen, as described by W. Duermeyer, J. v.d. Veen and B. Koster, Lancet 1978, I, 823.

## C. Preparation of F(ab')₂-fragments of IgG against hepatitis A

IgG was isolated from whole serum obtained from a hepatitis A patient by fractionation on DEAE-Sephadex in 0.0175 M phosphate buffer, pH 6.3.

A solution (1%—3%) of IgG in Walpole's acetate buffer (pH 4.3) was preheated to 37°C.

Pepsin, dissolved in the same buffer, was added to give an enzyme: substrate ratio of 1:100.

The mixture was incubated at 37°C for 20—24 hours.

The reaction was stopped by the addition of TRIS salt to adjust the pH to 8.

The fragments were separated on a Sephadex G 150 column in 0.1 M Tris/HCl, pH 7.7, +0.2 M NaCl, +2 mM EDTA.

## D. Preparation of enzyme-labelled F(ab')₂ conjugate

The F(ab')₂ conjugate against hepatitis A virus/antigen was prepared according to the method of P. K. Nakane and A. Kawaoi, J. Histochem. Cytoch., 22 (12), 1974, 1084—1091.

5 mg horse-radish peroxidase was dissolved in 1 ml 0.3 M carbonate buffer, pH 8.1 and 0.1 ml fluoro-dinitrobenzene, 1% in absolute alcohol, was added, followed by 1 ml 0.08 M sodium periodate.

The reaction was stopped with 1 ml 0.1 M ethylene glycol.

After dialysis against 0.01 M carbonate buffer, pH 9.5, 5 mg F(ab')₂ was added. This F(ab')₂ had previously been dialysed against 0.005 M PBS, pH 8.0.

After a reaction time of $2\frac{1}{2}$ hours at room temperature, the solution was dialysed overnight at 4°C against 0.005 M PBS, pH 8.0.

The conjugate was kept at 4°C.

## E. Determination of IgM antibodies against hepatitis A

The following test system was set up:

### I. Coating.

The wells of microtitration plates were coated by incubation with 5 µg IgG (anti-IgM) per ml in 0.05 M phosphate buffer pH 7.2 (PBS) for 16 hours at room temperature.

The plates were then washed 3× with PBS+0.05% Tween 20 (PBS/Tween).

### II. Test serum.

0.125 ml of a serum dilution in PBS/Tween was pipetted into the wells and incubated at 37°C for 4 hours, followed by washing 3× with PBS/Tween.

### III. Antigen.

0.1 ml antigen solution, in the working dilution, was added to each well. The plate was incubated overnight at room temperature, after which it was washed 4× with PBS/Tween.

### IV. Conjugate.

0.1 ml anti-hepatitis A—F(ab')₂ conjugate, diluted in PBS/Tween+5% negative human serum was pipetted into the wells, and the whole was incubated for 1 hour at 37°C, followed by washing 5× with PBS/Tween.

### V. Substrate

1 tablet ortho-phenylene diamine was dissolved in 30 ml distilled water (A).

1 tablet urea peroxide was dissolved in 7.5 ml distilled water (B).

0.5 ml (B) was added to 30 ml (A).

0.1 ml of this mixture was pipetted into each well and incubated in the dark for 45 minutes at room temperature.

The enzyme-substrate reaction was stopped with 0.05 ml 4N sulphuric acid.

Ortho-phenylene diamine and urea peroxide originated from a Hepanostika test kit for the determination of hepatitis B surface antigen, Organon Teknika, Oss, Holland.

### VI. Measurement of colour change.

The colour change of the substrate per well was measured at a wavelength of 492 nm, with the aid of a Vitatron photometer.

### VII. Interpretation.

5 negative controls were determined with each set of estimations. The mean extinction of these 5 controls+5× standard deviation served as the boundary between negative and positive sera.

## Example II

The interference of rheumatoid factor (RF) was investigated in a comparative test for the determination of anti-hepatitis A virus IgM antibodies (anti-HAV-IgM). A series of twenty RF positive sera, some of them containing also antihepatitis A virus IgG antibodies (anti-HAV-IgG), were tested with the test system as described in Example I.E., with the exception that besides the conjugate anti-HAV-F(ab')₂-HRP, as described in I.E.IV. also anti-HAV-Ig-HRP and anti-HAV-IgG-HRP are used in parallel tests. The latter conjugates have been prepared analogue to the method, described for anti-HAV-F(ab')₂-HRP in I.D.

No IgM antibodies were present in the test series, in which the F(ab')₂-HRP conjugate according to the invention is used. The Ig-HRP and IgG-HRP conjugates gave many false positive results.

Furthermore from the Ig fraction of this series of RF positive sera the IgG and IgM fractions have been isolated by sucrose gradient centrifugation and these fractions were tested separately in an anti-HAV-enzyme immuno-assay. It was confirmed, that no anti-HAV IgM antibodies were present.

The results are given in the table below. A sample was considered positive if the absorption, measured at 492 nm, after substrate addition, was higher than 2,1 times the absorbance of a negative control. The RF content is indicated with the reciprocal RF titre, determined according to Rose-Waaler's test.

| Serum | Reciprocal RF-titre (Rose-Waaler) | Result total anti-HAV-ELISA Sucrose fraction | | Result in anti-HAV-IgM test (ELISA) | | |
|---|---|---|---|---|---|---|
| | | IgG | IgM | Ig-HRP | IgG-HRP | F(ab')$_2$-HRP |
| 1 | 16 | − | − | − | − | − |
| 2 | 32 | + | − | − | + | − |
| 3 | 32 | − | − | − | − | − |
| 4 | 64 | + | − | − | + | − |
| 5 | 64 | + | − | + | + | − |
| 6 | 64 | + | − | − | + | − |
| 7 | 128 | − | − | − | + | − |
| 8 | 128 | − | − | − | + | − |
| 9 | 128 | + | − | − | + | − |
| 10 | 256 | + | − | + | + | − |
| 11 | 256 | + | − | − | + | − |
| 12 | 512 | + | − | + | + | − |
| 13 | 512 | + | − | + | + | − |
| 14 | 512 | − | − | − | + | − |
| 15 | 1024 | − | − | − | + | − |
| 16 | 1024 | + | − | + | + | − |
| 17 | 2048 | − | − | − | + | − |
| 18 | 2048 | − | − | + | + | − |
| 19 | 2048 | − | − | + | + | − |
| 20 | 2048 | + | − | + | + | − |
| Pos. control | | − | + | + | + | + |
| Neg. control 1 | | | | − | − | − |
| Neg. control 2 | | | | − | − | − |

Example III
Enzyme immuno assay for the detection and determination of human toxoplasma IgM-antibodies.

A. Preparation of anti-human IgM

A sheep was immunized with human IgM prepared from a Waldenström's macroglobulinaemia serum. The specificity of the antiserum was improved by absorption with human cord serum, which procedure removed the anti IgG-activity of the antiserum. 1 ml of sheep anti-human IgM serum was mixed with 0.5 ml human cord serum and incubated for 1 hour at 37°C. The mixture was centrifuged for 10 min. at 8,000 g. The supernatant was used as the anti-IgM serum; it was stored at 4°C.

B. Preparation of toxoplasma antigen

Toxoplasma gondii parasites were grown in the intraperitoneal cavity of mice for 3 days. The cells were harvested after killing the mice by rinsing the cavity with 1 ml phosphate-buffered saline (PBS) pH 7.2. A suspension of the cells in PBS was sonicated for 5 minutes with low intensity. Intact parasite cells were collected by centrifugation, resuspended in PBS pH 7.2 and sonicated with high intensity for 10 minutes. This suspension was used as an antigen preparation. It was frozen and stored at −20°C.

C. Preparation of fragments (F(ab')$_2$) of IgG directed against toxoplasma antigen.

A sheep was immunized with 1 mg of above mentioned toxoplasma antigen preparation. The serum was collected and the IgG was isolated by the caprilic acid method according to M. Steinbuch and R. Audran (Arch. Biochem. & Biophysics 134, 279—284 (1969)).

F(ab')$_2$-fragments were prepared by incubation of the IgG solution with pepsin in a 0.1 M acetate buffer pH 4,3 with a pepsin/IgG ratio of 1/50 for 8 hours at 37°C. The reaction was stopped by raising the pH to 8 by addition of sufficient solid tris(hydroxymethyl)-amino methane. The fragments were purified by gel-chromatography on a Sephacryl S 200 column in 0.1 M TRIS/HCI pH 7.5+0.2 M NaCl+0.002 M EDTA.

## D. Preparation of enzyme labelled F(ab')₂-fragment (conjugate)

Preparation of the F(ab')₂-conjugate was performed according to the method mentioned in Example I.D.

## E. Detection of IgM antibodies against toxoplasma gondii; assay procedure.

### I. Coating

The wells of polyvinylchloride microtitre plates were coated by adding a dilution of 1/10,000 of sheep anti human IgM serum in 0.05 M $Na_2CO_3$/HCI buffer at pH 9.0 and incubating for 16 hours at room temperature.

Hereafter the wells were washed three times with PBS (pH 7.2), containing 0.05% Tween 20 (PBS-Tween).

### II. Serum

Then 0.1 ml of an appropriate dilution of the test serum in PBS Tween was added and the wells were incubated for 4 hours at 37°C. Hereafter the wells were washed 3 times with PBS Tween.

### III. Antigen

The wells were incubated for 16 hours at room temperature with 0.1 ml of a suitable dilution of the antigen solution in PBS Tween. Hereafter the wells were washed 4 times with PBS Tween.

### IV. Conjugate

Then 0.1 ml of an appropriate dilution of anti-toxoplasma F(ab')₂-conjugate in PBS Tween was added and the wells were incubated for 1 hour at 37°C. Hereafter the wells were washed 5 times with PBS Tween.

### V. Substrate

The substrate solution was prepared acording to the method of Example I.V. 0.1 ml substrate solution was pipetted into each cup and incubated for 30 minutes at room temperature in the dark. The reaction was stopped by adding 0.1 ml of 4 N $H_2SO_4$ to the wells.

### VI. Measurement of colour change

The colour development in the wells was measured as the extinction of the substrate solution at 492 nm in a Vitatron photometer.

### VII. Interpretation of results

Together with each set of estimations 5 negative controls were tested simultaneously. A value of 2.1 times the mean extinction of these negative controls was taken as the boundary between positive and negative results.

## Example IV
## Detection of IgG antibodies against rubella virus/antigen

### A. Preparation of anti-human IgG serum

Rabbit anti-human IgG was prepared by injection of 0.5 mg purified human IgG into rabbits, followed by a booster injection of 0.5 mg IgG two months later. One week later the rabbits were bled and the serum was collected, and stored at —20°C until use.

### B. Preparation of rubella virus/antigen

Rubella virus M 33 strain was produced in BHK 21 cells cultured as a monolayer. Antigens were isolated by ultrasonication of the cells in a glycine/NaOH buffer pH 9.0. The suspension was centrifuged for 30 minutes at 3,000 g; the supernatant served as an antigen solution.

### C. Preparation of F(ab')₂-fragments of IgG aginst rubella

IgG was isolated from a human anti-rubella serum by caprilic acid precipitation of other serum-components. IgG in the supernatant was dialysed against 0.1 M NaAc/HAc buffer at pH 4.3. Pepsin was dissolved in the same buffer, and added to give a pepsin:IgG ratio of 1:50.

The mixture was incubated for eight hours at 37°C; the reaction was stopped by raising the pH to 8 with solid tris(hydroxymethyl)-amino methane. F(ab')₂-fragments were purified on a Sephacryl S 200 column in 0.1 M TRIS/HCI pH 7.5+0.9% NaCl.

### D. Preparation of enzyme-labelled F(ab')₂

F(ab')₂ conjugated was prepared according to the method described in Example I.D.

### E. Determination of IgG antibodies against rubella in human serum

### I. Coating

The wells of polystyrene microtitre plates were coated with F(ab')₂ fragments of rabbit-anti-human IgG (prepared from rabbit-anti-human IgG serum analogous to the preparation of F(ab')₂ fragments of IgG against Rubella in Example IV.C), by incubation of 0.1 ml F(ab')₂ solution in 0.05 M phosphate buffer, pH 7.2 (PBS) for 1 night at room temperature. After incubation the wells were washed with PBS+0.05% Tween 20 (pH 7.2).

### II. Test serum

0.1 ml of several dilutions of test serum in PBS Tween pH 7.2 were incubated for 2 hours at 37°C. Afterwards the wells were washed 3 times with PBS Tween (pH 7.2). Positive and negative control sera were tested simultaneously.

III. Antigen

0.1 ml of a dilution of the rubella antigen solution in PBS-Tween with pH 7.2 was incubated for 16 hours at room temperature. Afterwards the wells were washed 3 times with PBS-Tween (pH 7.2).

IV. Conjugate

0.1 ml F(ab')$_2$ anti-rubella conjugate, diluted in PBS Tween pH 7.2, was incubated for 1 hour at 37°C. Afterwards the wells were washed 5 times with PBS-Tween (pH 7.2).

V. Substrate

A solution of ortho-phenylene-diamine mixed with a solution of urea-peroxide was used as substrate. 0.1 ml of the substrate solution was incubated for 30 minutes at room temperature; the reaction was stopped by addition of 0.1 ml 4 N H$_2$SO$_4$.

VI. Measuring colour change

The change of colour in the wells was measured by measuring the extinction of the substrate solutions in the wells with a Vitatron photometer at a wavelength of 492 nm.

VII. Interpretation

The dilutions of the test sera that gave extinctions of 50% of the positive control were taken as the antibody titres of the sera.

"Sephadex", "Tween" and "Sephacryl" are registered Trade Marks.

**Claims**

1. Method for the detection and/or determination of an antigen specific immunoglobulin of a particular IgX class in which X means G, M, A, D or E, characterized in that the test medium is mixed with either insolubilized anti-IgX against the antigen specific immunoglobulin of a particular IgX class to be determined or antigen binding fragment of this anti-IgX with which the particular IgX to be determined is allowed to react, followed by an incubation with an antigen for which the immunoglobulin to be determined has specific affinity and subsequently with a labelled antigen binding fragment of an antibody against the above noted antigen, after which the labelling agent is detected and/or determined, which provides qualitative and/or quantitative information about the antigen specific immunoglobulin to be determined.

2. Method for the detection and/or determination of an antigen specific immunoglobulin of the IgM class, characterized in that the test medium is mixed with either insolubilized anti-IgM against the antigen specific immunoglobulin of the IgM class to be determined or antigen binding fragment of this anti-IgX with which the particular IgX to be determined is allowed to react, followed by an incubation with an antigen for which the immunoglobulin to be determined has specific affinity and subsequently with a labelled antigen binding fragment of an antibody against the above noted antigen, after which the labelling agent is detected and/or determined, which provides qualitative and/or quantitative information about the antigen specific immunoglobulin to be determined.

3. Method according to claim 1, characterized in that after mixing the test medium with the insolubilized anti-IgX or antigen binding fragment of this anti-IgX where the immunoglobulin to be determined is caused to be bound to the insolubilized anti-IgX component the insoluble phase is separated from the liquid phase.

4. Method according to claims 1 and 3, characterized in that after mixing of the test medium with an insolubilized anti-IgX or antigen binding fragment of this anti-IgX with which the particular IgX to be determined is allowed to react, a coupling product of the antigen and the labelled antigen binding fragment of the antibody is added to the test medium or the insolubilized phase.

5. Method according to claims 1—4, characterized in that the labelled antibody fragment is a labelled F(ab')$_2$ fragment.

6. Method according to claims 4—5, characterized in that the labelled antibody fragment is labelled with an enzyme.

7. Immuno-reagents, to be used in the method according to claims 4—6, comprising a coupling product of an antigen with a labelled antigen binding fragment of an antibody against the antigen concerned.

8. Test kit for the determination of an antigen specific immunoglobulin of a particular IgX class in which X means G, M, A, D or E, consisting predominantly of:

a) insolubilized anti-IgX against the antigen specific immunoglobulin of a particular IgX class to be determined or an antigen binding fragment of this anti-IgX,
b) an antigen, for which the immunoglobulin to be determined has a specific affinity,
c) a labelled antigen binding fragment of an antibody against said antigen.

9. Test kit for the determination of an antigen specific immunoglobulin of a particular IgX class in which X means G, M, A, D or E, consisting predominantly of:

a) insolubilized anti-IgX against the antigen specific immunoglobulin of a particular IgX class to be determined, or an antigen binding fragment of this anti-IgX,
b) a coupling product of an antigen, for which the immunoglobulin to be determined has specific affinity, with a labelled antigen binding fragment of an antibody against the antigen concerned.

10. Test kit according to claims 7—9, in which the labelling agent is an enzyme.

11. Test kit according to claim 10, which contains also a substrate for the enzyme concerned.

## Revendications

1. Procédé pour la détection et/ou la détermination d'une immunoglobuline à spécificité antigénique d'une classe IgX particulière où X signifie G, M, A, D ou E, caractérisé par le fait qu'on mélange le milieu étudié avec soit un anti-IgX insolubilisé dirigé contre l'immunoglobuline à spécificité antigénique d'une classe IgX particulière à déterminer, soit un fragment capable de liaison aux antigènes de cet anti-IgX avec lequel on laisse réagir l'IgX particulière à déterminer, puis on effectue une incubation avec un antigène pour lequel l'immunoglobuline à déterminer a une affinité spécifique puis avec un fragment marqué capable de liaison aux antigènes d'un anticorps contre l'antigène précité, après quoi on détecte et/ou détermine l'agent de marquage, ce qui fournit une information qualitative et/ou quantitative relative à l'immunoglobuline à spécificité antigénique à déterminer.

2. Procédé pour la détection et/ou la détermination d'une immunoglobuline à spécificité antigénique de la classe IgM caractérisé par le fait qu'on mélange le milieu étudié avec soit un anti-IgM insolubilisé dirigé contre l'immunoglobuline à spécificité antigènique de la classe IgM à déterminer soit avec un fragment insolubilisé capable de liaison aux antigènes de cet anti-IgX avec lequel on laisse réagir l'IgX particulière à déterminer, puis on effectue une incubation avec un antigène pour lequel l'immunoglobuline à déterminer a une affinité spécifique puis avec un fragment marqué capable de liaison aux antigènes d'un anticorps dirigé contre l'antigène de marquage, ce qui fournit une information qualitative et/ou quantitative relative à l'immunoglobuline à spécificité antigènique à déterminer.

3. Procédé selon la revendication 1, caractérisé par le fait qu'après le mélange du milieu étudié avec l'anti-IgX insolubilisé ou le fragment capable de liaison aux antigènes de cet anti-IgX, où l'immunoglobuline à déterminer est amenée à se lier au composant anti-IgX insolubilisé, on sépare la phase insoluble de la phase liquide.

4. Procédé selon les revendications 1 et 3, caractérisé par le fait qu'après le mélange du milieu étudié avec un anti-IgX insolubilisé ou un fragment capable de liaison aux antigènes de cet anti-IgX avec lequel on laisse réagir l'IgX particulier à déterminer, on ajoute un produit de couplage de l'antigène et du fragment marqué capable de liaison aux antigènes de l'anticorps au milieu d'essai ou à la phase insolubilisée.

5. Procédé selon l'une des revendications 1 à 4, caractérisé par le fait que le fragment d'anticorps marqué est un fragment F(ab')$_2$ marqué.

6. Procédé selon les revendications 4 et 5, caractérisé par le fait que le fragment d'anticorps maruqé est marqué avec une enzyme.

7. Immunoréactifs destinés à être utilisés dans le procédé selon l'une des revendications 4 à 6, comprenant un produit de couplage d'un antigène avec un fragment marqué capable de liaison aux antigènes d'un anticorps dirigé contre l'antigène concerné.

8. Nécessaire analytique pour la détermination d'une immunoglobuline à spécificité antigénique d'une classe IgX particulière où X signifie G, M, A, D ou E, constitué de façon prédominante de:

a) un anti-IgX insolubilisé dirigé contre l'immunoglobuline à spécificité antigénique d'une classe IgX particulière à déterminer ou un fragment capable de liaison aux antigènes de cet anti-IgX,
b) un antigène, pour lequel l'immunoglobuline à déterminer a une affinité spécifique,
c) un fragment marqué capable de liaison aux antigènes d'un anticorps dirigé contre ledit antigène.

9. Nécessaire analytique pour la détermination d'une immunoglobuline à spécificité antigénique d'une classe IgX particulière où X signifie G, M, A, D ou E, constitué de façon prédominante de:

a) un anti-IgX insolubilisé dirigé contre l'immunoglobuline à spécificité antigénique d'une classe IgX particulière à déterminer ou un fragment insolubilisé capable de liaison aux antigènes de cet anti-IgX,
b) un produit de couplage d'un antigène, pour lequel l'immunoglobuline à déterminer a une affinité spécifique, avec un fragment marqué capable de liaison aux antigènes d'un anticorps dirigé contre l'antigène concerné.

10. Nécessaire analytique selon l'une des revendications 7 à 9, dans lequel l'agent demarquage est une enzyme.

11. Nécessaire analytique selon la revendication 10, qui contient également un substrat pour l'enzyme concernée.

## Patentansprüche

1. Verfahren zum Nachweis und/oder zur Bestimmung eines antigenspezifischen Immunglobulins einer bestimmten IgX-Klasse, in der X G, M, A, D oder E bedeutet, dadurch gekennzeichnet, dass das Testmedium gemischt wird entweder mit unlöslich gemachtem Anti-IgX gegen das zu bestimmende, antigenspezifische Immunglobulin einer bestimmten IgX-Klasse oder mit antigenbindendem Fragment dieses Anti-IgXs, mit dem das zu bestimmende, spezielle IgX zur Reaktion gebracht wird, gefolgt durch eine Inkubierung mit einem Antigen, wofür das zu bestimmende Immunglobulin spezifische Affinität hat, und nachfolgend mit einem markierten antigenbindenden Fragment eines Antikörpers gegen das obenangegebene Antigen, worauf die Markierungssubstanz nachgewiesen und/oder bestimmt wird, was qualitative und/oder quantitative Information ergibt über das zu bestimmende antigespezifische Immunglobulin.

2. Verfahren zum Nachweis und/oder Bestimmung eines antigenspezifischen Immunglobulins der IgM-Klasse, dadurch gekennzeichnet dass das Testmedium gemischt wird entweder mit unlöslich gemachtem Anti-IgM gegen das zu bestimmende, antigenspezifische Immunglobulin der IgM-Klasse oder mit antigenbindendem Fragment dieses Anti-IgXs, mit dem das zu bestimmende, spezielle IgX zur Reaktion gebracht wird, gefolgt durch eine Inkubierung mit einem Antigen, wofür das zu bestimmende Immunglobulin spezifische Affinität hat, und nachfolgend mit einem markierten, antigenbindenden Fragment eines Antikörpers gegen das obenangegebene Antigen, worauf die Markierungssubstanz nachgewiesen und/oder bestimmt wird, was qualitative und/oder quantitative Information ergibt über das zu bestimmende antigenspezifische Immunglobulin.

3. Verfahren nach Anspruch 1, dadurch gekennzeichnet dass nach Mischung von dem Testmedium mit dem unlöslich gemachten Anti-IgX oder antigenbindendem Fragment dieses Anti-IgXs, wobei das zu bestimmende Immunglobulin veranlasst wird zur binden an die unlöslich gemachten Anti-IgX-komponente, die unlösliche Phase von der flussigen Phase getrennt wird.

4. Verfahren nach Ansprüche 1 und 3, dadurch gekennzeichnet, dass nach Mischung von dem Testmedium mit einem unlöslich gemachten Anti-IgX oder antigenbindendem Fragment dieses Anti-IgXs, mit dem das zu bestimmende, spezielle IgX zur Reaktion gebracht wird, ein Kupplungsprodukt von dem Antigen und dem markierten, antigenbindenden Fragment des Antikörpers zugefügt wird and das Testmedium oder die unlöslich gemachte Phase.

5. Verfahren nach Ansprüche 1—4, dadurch gekennzeichnet, dass das markierte Antikörperfragment ein markiertes F(ab')$_2$-fragment ist.

6. Verfahren nach Ansprüche 4—5, dadurch gekennzeichnet, dass das markierte Antikörperfragment markiert wird mit einem Enzym.

7. Immuno-Reagense zur Verwendung in dem Verfahren nach Ansprüche 4—6, enthaltend ein Kupplungsprodukt von einem Antigen mit einem markierten antigenbindenden Fragment eines Antikörpers gegen das betreffende Antigen.

8. Testpäckchen für die Bestimmung eines antigen-spezifischen Immunglobulins einer bestimmten IgX-Klasse, in der X G, M, A, D oder E bedeutet, hauptsächlich enthaltend:

a) unlöslich gemachtes Anti-IgX gegen das zu bestimmende antigenspezifische Immunglobulin einer bestimmten IgX-Klasse oder ein antigenbindendes Fragment dieses Anti-IgXs,
b) ein Antigen, wofür das zu bestimmende Immunglobulin eine spezifische Affinität hat,
c) ein markiertes antigenbindendes Fragment eines Antikörpers gegen genanntes Antigen.

9. Testpäckchen für die Bestimmung eines antigenspezifischen Immunglobulins einer bestimmten IgX-Klasse, in dem X G, M, A, D oder E bedeutet, hauptsäuchlich enthaltend:

a) unlöslich gemachtes Anti-IgX gegen das zu bestimmende, antigenspezifische Immunglobulin einer bestimmten IgX-Klasse oder ein antigenbindendes Fragment dieses Anti-IgX,
b) ein Kupplungsprodukt von einem Antigen, wofür das zu bestimmende Immunglobulin spezifische Affinität hat, mit einem markierten antigenbindenden Fragment eines Antikörpers gegen das betreffende Antigen.

10. Testpäckchen nach Ansprüche 7—9, in dem das markierende Agens eine Enzym ist.

11. Testpäckchen nach Anspruch 10, das zusätzlich ein Substrat für das betreffende Enzym enthält.